# EUROPEAN PATENT APPLICATION

(11) **EP 3 669 760 A1**
(43) Date of publication of application: **24.06.2020**
(21) Application number: 19152534.4
(22) Date of filing: 18.01.2019
(51) Int. Cl.: A61B 5/00, A61B 5/1455, A61B 5/1459, A61N 1/365, A61N 1/362, A61N 1/375

(54) **LEADLESS PACEMAKER OXYGEN-SATURATION-BASED RATE CONTROL**

(30) Priority: 18.12.2018 US 201862780960 P
(71) Applicant: BIOTRONIK SE & Co. KG, 12359 Berlin (DE)
(72) Inventor: SWENSON, Kurt, Dayton, OR 97114 (US); KRAETSCHMER, Hannes, West Linn, OR 97068 (US); TAFF, Brian M., Portland, OR 97217 (US)
(74) Representative: Engelhardt, Björn

(57) **Abstract**

The present disclosure relates to an implantable pacemaker (1), for applying electrical pacing pulses to a heart (H) of a patient, comprising: a pulse generator (2) configured to generate electrical pacing pulses comprising an adaptable rate, at least a first light source (10) configured to emit light comprising a first wavelength, at least a second light source (20) configured to emit light comprising a different second wavelength, a light receiving unit (30) arranged such with respect to the light sources (10, 20) that light emitted from the at least one first or the at least one second light source (10, 20) travels through blood (B) of the patient when the pacemaker (1) is implanted in the patient and impinges on the light receiving unit (30), which is configured to measure an intensity of light received from the at least one first or the at least one second light source (10, 20), and a processing unit (40) configured to determine an actual value of the ratio between a first value of an intensity of light emitted by the at least one first light source (10) and received by the light receiving unit (30) and a second value of an intensity of light emitted by the at least one second light source (20) and received by the light receiving unit (30).

## Description

The present invention relates to an implantable cardiac pacemaker, particularly to an implantable intracardiac (e.g. leadless) pacemaker.

The ultimate goal of the circulatory system of a human body is to provide adequate perfusion of the tissue within the body. The oxygen saturation level of the blood (the ratio of hemoglobin molecules that are carrying oxygen compared to ones that are not) is proportional to this goal, even in the right side of the heart where the blood is being returned to the lungs to be re-oxygenated. A known technique for measuring oxygen saturation is to use the differential in the light absorbed by the blood at different wavelengths. The minimum absorption by oxygenated hemoglobin occurs at a wavelength of about 660 nm, and the minimum absorption by deoxygenated hemoglobin occurs at a wavelength of about 940 nm.

The three central principles used for pacemaker rate response have typically been that a body in motion with particular characteristics is likely to be correlated with using the body's muscles for causing that motion, or that the hormonally controlled contractility of the heart can be used to tap into the body's intrinsic determination for cardiac demand, even if the electrically indicated heart rate indication from the body is not available, or to detect a condition that correlates with cardiac demand.

Rate response in pacemakers has been done using motion (in both leadless and conventional pacemakers), cardiac contractility (in conventional pacemakers), cardiac temperature (in both leadless and conventional pacemakers), and volume change of breathing (in conventional pacemakers).

Motion sensing in leadless pacemakers is complicated by the fact that the heart motion can be difficult to differentiate from the body motion. Also, because of the nature of how leadless pacemakers are affixed in the heart, the pacemaker may be positioned in almost any orientation compared to the main axis of the body, and the cardiac motion can change this orientation dynamically. Motion sensors are axis specific.

Furthermore, cardiac temperature, while loosely associated with cardiac demand, is affected by sweating, environmental conditions and fevers as well.

The commonly used indicators for rate demand are actually only proxies for what really matters, which is energy efficient perfusion of the body tissue by the blood. Such proxies, while they may be statistically representative of the rate demand level, are not always accurate. For example, motion signals from going down stairs typically involve more vigorous accelerations than the signals from going up stairs, but the cardiac demand associated with going up stairs is almost always greater than with going down stairs. Inferring the autonomic nervous system cardiac demand rate from contractility or breathing measurements is also sometimes sensitive to possibly unrelated changing conditions like orthostasis.

Document US 2016/0338629 A1 describes a device that is configured to be inserted into the human or animal body, wherein the device includes at least one sensor unit that detects a signal representative of an oxygen content along a measurement path, wherein the sensor may include a reflector provided on the device, wherein the reflector is arranged in the measurement path between a transmitter and a receiver of the sensor to reflect the signal.

Any approach that relies on measuring a condition in the heart can potentially be done using a special lead with a sensor that is located at the distal end of the lead. However, pacemaker systems that use special leads are not always desirable since they may increase complexity of the whole system.

Based on the above it is an objective of the present invention to provide a pacemaker that can offer a more flexible and more physiological pacing rate control that can in particular be achieved independent of placement orientation of the device and variations in cardiac motion characteristics.

An implantable cardiac pacemaker according to claim 1 is provided. Further embodiments are subject matter of dependent claims.

Therefore, an implantable cardiac pacemaker is disclosed that is configured to apply electrical pacing pulses to a heart of a patient, wherein the pacemaker comprises:
- a pulse generator configured to generate electrical pacing pulses comprising an adaptable rate,
- at least a first light source configured to emit light comprising a first wavelength,
- at least a second light source configured to emit light comprising a second wavelength, wherein the second wavelength is different from the first wavelength,
- a light receiving unit arranged such with respect to the light sources that light emitted from the at least one first light source or the at least one second light source travels through blood of the patient when the pacemaker is implanted in the patient and impinges on the light receiving unit, which is configured to measure an intensity of light received from the at least one first light source or the at least one second light source, and
- a processing unit configured to determine an actual value of a ratio or a difference between a first value of an intensity of light emitted by the at least one first light source and received by the light receiving unit and a second value of an intensity of light emitted by the at least one second light source and received by the light receiving unit.

Particularly, said ratio or said difference is indicative of an oxygen content in the blood of the patient.

Particularly, the at least one first light source can be formed by an LED (LED - light-emitting diode) or an OLED (OLED - organic light-emitting diode). Further, particularly, the at least one second light source can be formed by an LED or an OLED, too.

Furthermore, the light receiving unit can comprise a single photodetector (e.g. a photodiode or a phototransistor) which is responsive to both the first wavelength and the second wavelength. Alternatively, the light receiving unit can comprise a first photodetector and a second photodetector (or multiple photodetectors, see below), particularly such that light emitted from the first light source travels through blood of the patient when the pacemaker is implanted in the patient and impinges on the first photodetector, which is configured to measure an intensity of light received from the first light source, and such that light emitted from the second light source travels through blood of the patient when the pacemaker is implanted in the patient and impinges on the second photodetector, which is configured to measure an intensity of light received from the second light source.

According to a preferred embodiment, the pacemaker does not comprise a dedicated reflector configured to reflect or deflect light from the first light source or the second light source on its way to the light receiving unit. Particularly, according to an embodiment, the light receiving unit faces the at least one first light source in a direction in which the at least one first light source emits light towards the light receiving unit. Further, in a corresponding embodiment, the light receiving unit faces the at least one second light source in a direction in which the at least one second light source emits light towards the light receiving unit. Alternatively, the light receiving unit and the first and second light sources are arranged side by side along a surface of a casing of the pacemaker. A portion of the light transmitted into the bloodstream will be scattered by the blood, while another portion of the light will be absorbed by molecules in the blood. Some of the scattered light will be returned in the direction of the implant and the amount of scatter return to the light receiving unit will be directly related to how much is absorbed. The surrounding tissue is mostly transparent to light at these wavelengths.

According to a further embodiment of the cardiac pacemaker, the processing unit is configured to form said actual value of the ratio or the difference by calculating said ratio or said difference for a pre-defined number of cardiac cycles and averaging said ratio or said difference over said number of cardiac cycles. A cardiac cycle is typically a heartbeat. Also, a ventricular extrasystole may be considered a separate cardiac cycle.

Particularly, according to an embodiment of the cardiac pacemaker, the pacemaker is configured to control the rate of the electrical pacing pulses in an open-loop manner, wherein a particular value of said ratio or said difference corresponds to a particular target rate. However, closed-loop control is preferred in the framework of the present disclosure, see also below.

Furthermore, according to an embodiment of the cardiac pacemaker, the light emitted from the at least one first light source comprises a wavelength in the range from 640 nm to 680 nm. Further, in an embodiment, light emitted from the at least one second light source comprises a wavelength in the range from 920 nm to 1050 nm. Particularly, the wavelength of the light emitted from the at least one first light source corresponds to 660 nm. Furthermore, particularly, the wavelength of the light emitted from the at least one second light source corresponds to 940 nm. These wavelengths may also apply to embodiments using multiple first light sources (e.g. LEDs or OLEDs) and multiple second light sources (e.g. LEDs or OLEDs).

Furthermore, according to an embodiment, the pacemaker (particularly the processing unit) is configured to alternately turn the at least one first light source and the at least one second light source on and off, so that at a (first) activation time at which the at least one first light source is turned on in order to emit light, the at least one second light source is turned off, and such that at a (second) activation time at which the at least one second light source is turned on in order to emit light, the at least one first light source is turned off.

Furthermore, according to an embodiment, the cardiac pacemaker is configured to synchronize said activation times at which the at least one first light source or the at least one second light source emits light with an electrical activity of a chamber of a heart of the patient (e.g. the right ventricle, the left ventricle, the right atrium, or the left atrium). Particularly, said electrical activity indicates a contraction of the considered chamber. The synchronization can be performed with a phasing.

Particularly, in an embodiment, the pacemaker is configured to synchronize said activation times relative to a timing of the electrical pacing pulses or relative to a timing at which a sensor of the pacemaker detects an electrical activity in the chamber (indicating a contraction of the chamber).

Furthermore, according to an embodiment of the cardiac pacemaker, the pacemaker (particularly the processing unit) is configured to regularly interchange the activation times at which the at least one first light source and the at least one second light source are turned on, particularly so as to cancel out the slight difference due to the particular activation times of the light sources.

Furthermore, according to an embodiment, the pacemaker comprises a constant current source (e.g. a battery) for driving the respective light source as well as a means for switching the current applied to the respective light source on and off. Particularly, the timing sequences for the measurements of said ratio or said difference and the search algorithm described herein can be provided by the processing unit which may comprise a microprocessor or a dedicated logic state machine.

Furthermore, according to an embodiment of the pacemaker, said first value is a digital value, namely a first digital value, and said second value is a digital value, namely a second digital value, wherein the pacemaker (particularly the processing unit) comprises an analog-to-digital converter (ADC) to obtain the first digital value by converting an analog signal provided by the light receiving unit that is indicative of the intensity of the light received from the at least one first light source, and to obtain the second digital value by converting an analog signal provided by the light receiving unit that is indicative of the intensity of the light received from the at least one second light source.

Furthermore, according to an embodiment of the pacemaker, the pacemaker (particularly the processing unit) is configured to convert said analog signal indicative of the intensity of the light received from the at least one first light source into the first digital value while the at least one first light source emits light. Likewise, the pacemaker (particularly the processing unit) is particularly configured to convert said analog signal indicative of the intensity of the light received from the at least one second light source into the second digital value while the at least one second light source emits light.

Furthermore, according to a preferred embodiment, the cardiac pacemaker (particularly the processing unit) is configured to control said ratio or said difference in a closed-loop fashion by adjusting the rate of the pacing pulses to achieve a desired ratio or a desired difference (particularly so as to maximize said ratio or said difference which are indicative of an oxygen saturation of the blood of the patient).

Furthermore, according to a preferred embodiment, the pacemaker is configured to generate and apply pacing pulses with a first rate and with a second rate, wherein the second rate is higher than the first rate, wherein the pacemaker (particularly the processing unit) is configured to calculate said ratio or said difference for the first rate and for the second rate, wherein the pacemaker is configured to increase the first rate and the second rate in case the ratio or the difference calculated for the second rate exceeds the ratio or the difference for the first rate by a pre-defined amount, and wherein in case the ratio or the difference calculated for the second rate does not exceed the ratio or the difference calculated for the first rate by a pre-defined amount, the pacemaker is configured to decrease the first rate and the second rate.

Particularly, when the average rate being used is near the optimum rate, the measurement rates will alternate between a slightly higher rate pair (where there is no significant difference seen) and a slightly lower rate pair (where some difference in saturation is detected).

Furthermore, according to an embodiment of the cardiac pacemaker, in order to decrease the time to find an optimum rate of the pacing pulses provided by the pacemaker, the pacemaker is configured to increase the first rate and the second rate by a first rate increment as a search progresses towards higher first and second rate pairs, and wherein in case the search backtracks to a lower rate pair, the pacemaker is configured to use a second rate increment that is smaller than the first rate increment.

Furthermore, according to a preferred embodiment, the pacemaker comprises a casing comprising a circumferential lateral surface, wherein the light receiving unit comprises at least one photodetector, wherein the at least one first light source and the at least one second light source are arranged along the surface in a circumferential direction of the casing. Furthermore, according to a preferred embodiment, the at least one photodetector is arranged in the circumferential direction between the at least one first light source and the at least one second light source, such that light emitted from the at least one first light source or the at least one second light source travels through blood of the patient when the pacemaker is implanted in the patient, is backscattered by the blood of the patient and impinges on the at least one photodetector of the light receiving unit, which is configured to measure an intensity of light received from the at least one first light source or the at least one second light source.

Furthermore, according to a further embodiment, multiple first and second light sources can be used. Here, particularly, the pacemaker comprises a casing comprising a circumferential lateral surface, wherein the light receiving unit comprises a plurality of photodetectors, and wherein the cardiac pacemaker comprises a plurality of first light sources configured to emit light comprising the first wavelength, and a plurality of second light sources configured to emit light comprising the second wavelength, wherein the first and the second light sources are arranged in an alternating manner along the surface in a circumferential direction of the casing.

Furthermore, according to an embodiment, a photodetector is arranged in the circumferential direction between each first and neighboring second light source, such that light emitted from the first or second light sources travels through blood of the patient when the pacemaker is implanted in the patient, is backscattered by blood of the patient and impinges on the photodetectors of the light receiving unit that are arranged between the light sources. Further, the light receiving unit is particularly configured to measure an intensity of light received from the first or second light sources, and the processing unit is configured to determine an actual value of the ratio or the difference between a first value of an intensity of light emitted by the first light sources and received by the light receiving unit and a second value of an intensity of light emitted by the second light sources and received by the light receiving unit.

Further, according to another embodiment of the pacemaker, the pacemaker comprises a casing through which a flow channel for guiding a blood stream extends, wherein the light receiving unit and the light sources are integrated in the casing, and wherein the flow channel is arranged between the light receiving unit and the at least one first light source and the at least one second light source, such that light emitted by the respective light source travels across the flow channel and impinges on the light receiving unit.

Furthermore, according to yet another preferred embodiment of the present invention, the cardiac pacemaker is an intracardiac pacemaker, wherein the pulse generator is enclosed by the casing.

Furthermore, in an embodiment, the casing is configured to be anchored to heart tissue of the patient, when the intracardiac pacemaker is implanted in the heart of a patient, wherein particularly the intracardiac pacemaker is configured to be implanted in an atrium or a ventricle of the patient. Particularly, the intracardiac pacemaker can comprise multiple tines for anchoring the casing to heart tissue of the patient. The tines can be arranged at a distal end of the casing.

Furthermore, according to an embodiment, the intracardiac pacemaker comprises a pacing electrode for applying the pacing pulses to the heart of the patient, wherein the pacing electrode is arranged on the casing, e.g. on the distal end of the casing (such a pacemaker is also denoted as implantable leadless pacemaker). The intracardiac pacemaker may also comprise a return electrode. The return electrode may be formed as a ring electrode which may be arranged at a proximal region of the casing.

According to an alternative embodiment, the cardiac pacemaker can comprise a housing which encloses the pulse generator, which housing is configured to be implanted in the patient outside the heart of the patient, wherein the cardiac pacemaker comprises a flexible lead extending from the housing, which lead forms said casing.

In the following embodiments, features and advantages of the present invention shall be explained with reference to the Figures, wherein
- Fig. 1A: shows cross section through the device of Fig. 1B along the lines A -A,
- Fig. 1B: shows an illustration of an embodiment of an intracardiac pacemaker comprising light sources and a light receiving unit/photodetectors arranged on a periphery of a casing of the pacemaker such that optical signals that pass through the blood, some of which are returned to the light receiving unit as a result of scattering, report oxygenation conditions to the light receiving unit of the pacemaker; and
- Fig. 2: shows an illustration of an alternative embodiment of an intracardiac pacemaker comprising a casing having a flow channel into which a light receiving unit and light sources are incorporated.

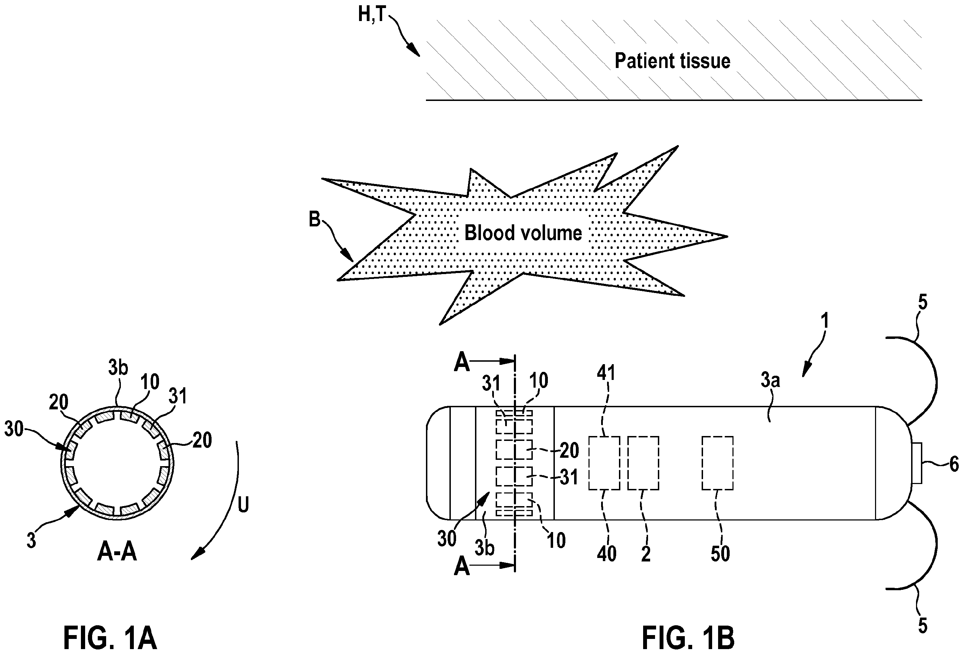

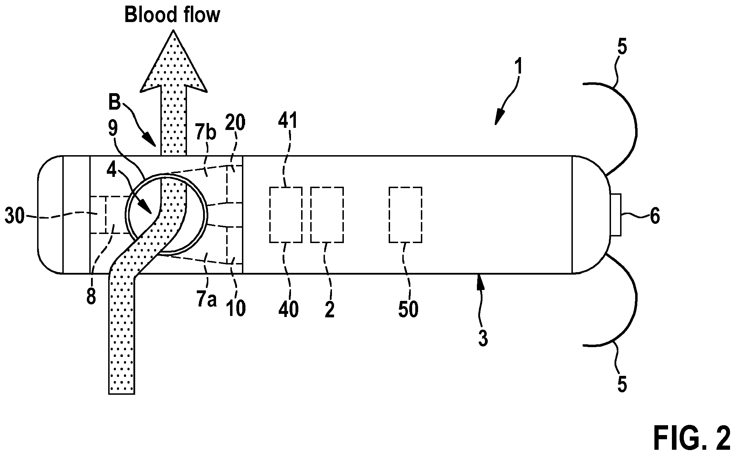

The present disclosure relates to a cardiac pacemaker 1 as shown e.g. in Figs. 1A, 1B and 2. According thereto, the pacemaker 1 comprises at least a pulse generator 2 configured to generate electrical pacing pulses comprising an adaptable rate, at least a first light source 10 configured to emit light comprising a first wavelength, at least a second light source 20 configured to emit light comprising a second wavelength which is different from the first wavelength, a light receiving unit 30 arranged such with respect to the light sources 10, 20 that light emitted from the at least one first or the at least one second light source 10, 20 travels through blood B of the patient when the pacemaker 1 is implanted in the patient and impinges on the light receiving unit 30, which is configured to measure an intensity of light received from the at least one first or the at least one second light source 10, 20. Furthermore, the pacemaker 1 comprises a processing unit 40 configured to determine an actual value of a ratio or a difference between a first value of an intensity of light emitted by the at least one first light source 10 and received by the light receiving unit 30 and a second value of an intensity of light emitted by the at least one second light source 20 and received by the light receiving unit 30. The pulse generator 2, the processing unit 40 and a battery 50 may be arranged in a casing 3. The battery 50 may be configured to provide electrical energy to the components of the pacemaker 1, in particular to the processing unit 40, the at least one first light source 10, the at least one second light source 20 and/or the light receiving unit 30.

The light sources 10, 20 are preferably formed by LEDs or OLEDs, and are preferably used for differentiating the absorption of their light between oxygenated and deoxygenated hemoglobin cells in the blood B around the implanted pacemaker 1. The light sources 10, 20 are configured such that they will illuminate the bloodstream B within the cardiac chamber where they are located when activated. The light receiving unit 30 is arranged in a manner that it will see the light from these (O)LEDs 10, 20 after it has passed through some of the blood flow B, facilitating a direct reading of oxygen content within the blood B rather than an extracted assessment from the more "downstream" conditions associated with perfused tissue.

According to the embodiment shown in Fig. 2, a flow channel 4 is provided extending through the casing 3 of the pacemaker, such that blood B can pass between the (O)LEDs 10, 20 and the light receiving unit 30. Alternatively, as shown in Fig. 2, light backscattered in the blood stream B can be captured by the light receiving unit 30.

As shown in detail in Fig. 2, the flow channel 4 can extend in a radial direction of the casing through the casing 3 so that the flow channel 4 connects two surface portions of the casing 3 that face away from each other. Particularly, the light receiving unit 30 and the light sources 10, 20 are integrated in the casing 3 such that the flow channel 4 is arranged between the light receiving unit 30 on one side and the at least one first light source 10 and the at least one second light source 20 on the other side. In an embodiment, the receiving unit 30 can be optically coupled to the flow channel 4 via a first light pipe 8 that extends from the light receiving unit 30 and meets the flow channel 4. Furthermore, in an embodiment, the at least one first light source 10 can be optically coupled to the flow channel 4 via a second light pipe 7a that extends from the at least one first light source 10 and meets the flow channel 4. In the same manner, according to an embodiment, the at least one second light source 20 can be optically coupled to the flow channel 4 via a third light pipe 7b, too. Here, the third light pipe 7b extends from the at least one second light source 20 and meets the flow channel 4. In an embodiment, the flow channel 4 can be delimited by a tubing 9 having a transparent wall. The tubing 9 serves for separating the blood stream B from the unit 30 and light sources 10, 20.

Particularly, as indicated in Figs. 1B and 2, the respective pacemaker 1 preferably is an intracardiac pacemaker 1, i.e. the casing 3 which comprises the pulse generator 2 and a pacing electrode 6 arranged on the casing 3 (e.g. at a distal end of the casing 3) is configured to be implanted in a chamber of the heart H of the patient (e.g. in a ventricle or in an atrium of the patient). Particularly, the casing 3 is configured to be anchored to heart tissue T of the patient, e.g. using multiple (e.g. three or four) tines 5 protruding from the casing 3.

In contrast to Fig. 2, the embodiment shown in Fig. 1A and 1B does not need a dedicated flow channel 4 integrated into the casing 3 of the pacemaker 1 and therefore comprises a simpler design. Here, photodetectors 31 of the light receiving unit 30 as well as first and second light sources (e.g. LEDs or OLEDs) 10, 20 are arranged in a circumferential direction U along a lateral surface 3a of the (e.g. cylindrical) casing 3. The first and second light sources 10, 20 are arranged in an alternating manner side by side in the circumferential direction, wherein photodetectors 31 are arranged between each two neighboring light sources 10, 20. In one embodiment, a single first light source 10, a single second light source 20, and a single photodetector 31 are provided. Also here, according to an embodiment, the lateral surface 3a comprises a tubing 3b having a transparent wall, so that light can be transmitted and received through the tubing 3b.

Both designs shown in Figs. 1B and 2 can be modified by positioning the optical monitoring portion of the device at the distal tip of a tail extension as well (not specifically drawn). The optical parts, either the LEDs or the photo sensor or both, could be located in a tail extension to either position them in a better spot in the heart or to juxtapose them to increase the optical coupling between them. This could utilize either the direct optical path approach (i.e. LEDs face sensor with blood flow between them) or the scatter return approach (i.e. LEDs and sensor point into the bloodstream).

According to an embodiment, the light sources / (O)LEDs 10, 20 shown in Figs. 1A, 1B and 2 are configured to be pulsed on separately, but close together in time such that they are emitting light under similar conditions. The measurements are preferably conducted in a synchronous fashion and/or phase shifted to a time of stable flow to minimize errors due to changes during the measurement period with respect to the chamber activity of the patient by using either the pace timing or the sense timing made by the pacemaker 1. To account for the slight difference due to the particular activation times of the light sources / (O)LEDs 10, 20, the order can be swapped every other time so that the differences can be cancelled out. The ratio or the difference between the levels of sensed light for the two different light sources 10, 20 are preferably calculated and averaged over several cardiac cycles.

The (e.g. average) ratio of the intensity of light measured due to the lower absorption from the ∼660 nm light (oxygenated hemoglobin) of the first light source 10 divided by the intensity of the light measured due to the lower absorption from the ∼940 nm light (deoxygenated hemoglobin) of the second light source 20 can be used as a control variable in a control loop that searches the rate space over time to find the minimum rate that maximizes this ratio (i.e. that maximizes oxygen saturation of the blood in the chamber in question). This rate can be continuously searched as long as rate adaptation is desired.

Particularly, the configuration for making the measurements includes at least the two light sources 10, 20 (e.g. LEDs or OLEDs), wherein constant current sources may be used for driving the (O)LEDs 10, 20. A switching means can be used for switching the respective current on and off. The timing sequences for the measurements and the search algorithm described below can be implemented into the processing unit 40, which may comprise either a microprocessor or a dedicated logic state machine. Particularly, regarding the embodiment shown in Fig. 1A and 1B, a single photodetector 30 which is responsive in both the 940 nm and 660 nm wavelengths can be used to convert the reflected light into an analog signal which is converted to a digital value using an analog-to-digital converter (ADC) 41.

The conversions are preferably timed to occur while the respective light source 10, 20 is illuminated. The converted signals from the two light sources activations are compared by dividing one by the other for forming the ratio defined above. Alternatively, one of the measurements can be subtracted from the other to obtain a difference rather than a ratio. The difference can be used in the same way as the ratio. Subtractions typically require less power to compute, but will be more susceptible to errors that might result due to differences in the photodetector response to the two wavelengths being used. The use of two different photodetectors to measure the different wavelengths is also conceivable and may improve resolution by providing better matched responses, but would take up more installation space. If necessary, the differences in response can be dealt with by means of proper calibration.

In order to adapt the rate of the pacing pulses applied by the pacemaker based e.g. on said calculated ratio or said difference, an open-loop control system may be used, where a particular saturation level would correspond with a particular target rate, although a closed-loop control system has significant advantages.

The closed-loop approach allows for adapting to variations in the cardiac environment, adapting to variations in the performance characteristics of the light sources 10, 20 (e.g LEDs or OLEDs) and the light receiving unit 30, variations in the light transmission and reflectance in the path that the light takes from the light sources 10, 20 to the light receiving unit 30, and can provide a more robust support in patients that may not be able to achieve 100% saturation levels. Typically, it can be expected that once the rate which corresponds with a 100% saturation level is found, that any rate greater than this will correspond with a 100% saturation level as well. The higher rates will be potentially detrimental to the patient since more biologically-derived energy will be used with no additional benefit. The closed-loop approach is amenable to a secondary search strategy that finds the lowest rate which gives the highest saturation level.

In the closed-loop approach, the measurements are preferably made under the condition of two close but separate pacing rates. The difference in the measurements (each measurement consisting of a comparison between the response at the two wavelengths as described above) becomes the feedback signal used to determine the next two rates that will be tried. For stability of the control loop, a threshold can be applied to the difference between the measurements to determine whether it is significant. Also, the measurements can be averaged over some time period (e.g. several heart beats), potentially synchronized with the cardiac activity, in order to reduce random and systemic variations that are not related to a variation in blood oxygen saturation. When the measurement from the higher rate gives a significantly higher saturation indication than the lower rate, the two rates for the next measurements will be increased. When the measurement from the higher rate does not show a significantly higher indication of saturation, the two rates for the next measurements will be decreased. When the average rate being used is near the optimum rate, the measurement rates will alternate between a slightly higher rate pair (where there is no significant difference seen) and a slightly lower rate pair (where some difference in saturation is detected).

In order to decrease the time to find the optimum rate when the starting rate is not close, the rate increment used when searching from a lower rate pair to higher rate pairs could be systematically increased as the search progresses in the upward direction. Once the search backtracks to a lower rate pair, the increment would be restored to the initial smaller increment.

The closed loop search approach has the advantage of robustly adapting to different patient measurements, but the energy use can be costly. An enhancement might be to use the search to build up characterization data for the individual patient's measurements and then use that data to run in a more energy efficient open loop mode for a time period in which the characterization data can be expected to be stable.

The present invention advantageously allows to using oximetry to determine the oxygen perfusion level being achieved by the circulatory system. This is possible by facilitating an assessment of oxygenation via direct measurements within the blood volume as opposed to measuring perfusion within the patient's tissue, a process that can be expected to provide results with a significantly longer delay time. Particularly, the present invention can be applied to leadless pacemaker systems as exemplary shown in Figs. 1A, 1B and 2, wherein the pacing rate space can be efficiently searched to find the lowest rate that optimizes the calculated perfusion level. For this, the present invention particularly may employ a closed-loop control algorithm to adapt the system to changing conditions and to accommodate variations between different patients. Therefore, the approach can offer a more physiological pacing rate control than existing pacemaker rate response algorithms. It can be achieved in a leadless/intracardiac pacemaker independent of placement orientation and variations in cardiac motion characteristics. As opposed to an open-loop rate control, it may adapt to changing conditions and variations between patients.

## Claims

1. An implantable pacemaker (1), for applying electrical pacing pulses to a heart (H) of a patient, comprising:
- a pulse generator (2) configured to generate electrical pacing pulses comprising an adaptable rate,
- a first light source (10) configured to emit light comprising a first wavelength,
- a second light source (20) configured to emit light comprising a second wavelength, wherein the second wavelength is different from the first wavelength,
- a light receiving unit (30) arranged such with respect to the light sources (10, 20) that light emitted from the first light source (10) or the second light source (20) travels through blood (B) of the patient when the pacemaker (1) is implanted in the patient and impinges on the light receiving unit (30), which is configured to measure an intensity of light received from the first light source (10) or the second light source (20), and
- a processing unit (40) configured to determine an actual value of a ratio or a difference between a first value of an intensity of light emitted by the first light source (10) and received by the light receiving unit (30) and a second value of an intensity of light emitted by the second light source (20) and received by the light receiving unit (30).

2. The cardiac pacemaker (1) according to claim 1, wherein the processing unit (40) is configured to form said actual value of the ratio or the difference by calculating said ratio or said difference for a pre-defined number of cardiac cycles and averaging the ratios or differences over said number of cardiac cycles.

3. The cardiac pacemaker (1) according to one of the preceding claims, wherein the light emitted from the first light source (10) comprises a wavelength in the range from 640 nm to 680 nm, and/or wherein light emitted from the second light source (20) comprises a wavelength in the range from 920 nm to 1050 nm.

4. The cardiac pacemaker (1) according to one of the preceding claims, wherein the pacemaker (1) is configured to alternately turn the first light source (10) and the second light source (20) on and off, so that at an activation time at which the first light source (10) is turned on in order to emit light, the second light source (20) is turned off, and such that at an activation time at which the second light source (20) is turned on in order to emit light, the first light source (10) is turned off.

5. The cardiac pacemaker (1) according to claim 4, wherein the cardiac pacemaker (1) is configured to synchronize said activation times at which the first light source (10) or the second light source (20) emits light with an electrical activity of a chamber of the heart (H) of the patient.

6. The cardiac pacemaker (1) according to claim 4 or 5, wherein the pacemaker (1) is configured to regularly swap the activation times at which the first light source (10) and the second light source (20) are turned on.

7. The cardiac pacemaker (1) according to one of the preceding claims, wherein said first value is a digital value, namely a first digital value, and wherein said second value is a digital value, namely a second digital value, wherein the pacemaker (1) comprises an analog-to-digital converter (41) to obtain the first digital value by converting an analog signal provided by the light receiving unit (30) that is indicative of the intensity of the light received from the first light source (10), and to obtain the second digital value by converting an analog signal provided by the light receiving unit (30) that is indicative of the intensity of the light received from the second light source (20).

8. The cardiac pacemaker (1) according to claim 7, wherein the pacemaker (1) is configured to convert said analog signal indicative of the intensity of the light received from the first light source (10) into the first digital value while the first light source (10) emits light, and/or wherein the pacemaker (1) is configured to convert said analog signal indicative of the intensity of the light received from the second light source (20) into the second digital value while the second light source (20) emits light.

9. The cardiac pacemaker (1) according to one of the preceding claims, wherein the cardiac pacemaker (1) is configured to control said ratio or said difference by adjusting the rate of the pacing pulses to achieve a desired ratio or a desired difference.

10. The cardiac pacemaker (1) according to one of the preceding claims, wherein the pacemaker (1) is configured to generate pacing pulses with a first rate and with a higher second rate, wherein the pacemaker (1) is configured to calculate said ratio or said difference for the first rate and for the second rate, wherein the pacemaker (1) is configured to increase the first rate and the second rate in case the ratio or the difference calculated for the second rate exceeds the ratio or the difference for the first rate by a pre-defined amount, and wherein in case the ratio or the difference calculated for the second rate does not exceed the ratio or the difference calculated for the first rate by a pre-defined amount, the pacemaker (1) is configured to decrease the first rate and the second rate.

11. The cardiac pacemaker (1) according to claim 10, wherein in order to decrease the time to find an optimum rate of the pacing pulses, the pacemaker (1) is configured to increase the first rate and the second rate by a first rate increment as a search progresses towards higher first and second rate pairs, and wherein in case the search backtracks to a lower rate pair, the pacemaker (1) is configured to use a second rate increment that is smaller than the first rate increment.

12. The cardiac pacemaker (1) according to one of the preceding claims, wherein the pacemaker (1) comprises a casing (3) comprising a circumferential lateral surface (3a), wherein the light receiving unit (30) comprises a plurality of photodetectors (31), and wherein the pacemaker (1) comprise a plurality of first light sources (10) configured to emit light comprising the first wavelength, and a plurality of second light sources (20) configured to emit light comprising the second wavelength, wherein the first and the second light sources (10, 20) are arranged in an alternating manner along the lateral surface (3a) in a circumferential direction (U) of the casing (3), and wherein a photodetector (31) is arranged in the circumferential direction (U) between each first and neighboring second light source (10, 20), such that light emitted from the first or second light sources (10, 20) travels through blood (B) of the patient when the pacemaker (1) is implanted in the patient, is backscattered by the blood (B) of the patient and impinges on the photodetectors (31) of the light receiving unit (30), which is configured to measure an intensity of light received from the first or second light sources (10, 20), and wherein the processing unit (40) is configured to determine an actual value of the ratio or the difference between a first value of an intensity of light emitted by the first light sources (10) and received by the light receiving unit (30) and a second value of an intensity of light emitted by the second light sources (20) and received by the light receiving unit (30).

13. The cardiac pacemaker (1) according to one of the claims 1 to 11, wherein the pacemaker comprises a casing (3) through which a flow channel (4) for guiding a blood stream (B) extends, wherein the light receiving unit (30) and the light sources (10, 20) are integrated in the casing (3), and wherein the flow channel (4) is arranged between the light receiving unit (30) and the first light source (10) and the second light source (20), such that light emitted by the respective light source (10, 20) travels across the flow channel (4) and impinges on the light receiving unit (30).

14. The cardiac pacemaker (1) according to claim 12 or 13, wherein the pacemaker (1) is an intracardiac pacemaker, wherein the pulse generator (2) is enclosed by the casing (3).
